# EUROPEAN PATENT APPLICATION

(11) **EP 4 789 655 A1**
(43) Date of publication of application: **12.08.2026**
(21) Application number: 25878448.7
(22) Date of filing: 22.01.2025
(51) Int. Cl.: A61K 8/02, A61Q 1/12, A61Q 19/00, F26B 5/06, B05D 5/06, B05D 7/22

(54) **PREPARATION METHOD FOR COSMETIC POWDER BLOCK HAVING DOT-STAINING EFFECT, AND COSMETIC POWDER BLOCK**

(30) Priority: 02.12.2024 CN 202411763231
(71) Applicant: A & H International Cosmetics Co., Ltd, Shanghai 201415 (CN)
(72) Inventor: TIAN, Yong, Shanghai 201415 (CN); CHEN, Jinhai, Shanghai 201415 (CN); YANG, Yongxiang, Shanghai 201415 (CN); LIU, Qingyu, Shanghai 201415 (CN); WU, Yidi, Shanghai 201415 (CN); SHEN, Jie, Shanghai 201415 (CN); SHIN, Bokchul, Shanghai 201415 (CN); ZHANG, Jinnv, Shanghai 201415 (CN); TIAN, Yuncai, Shanghai 201415 (CN)
(74) Representative: Cohausz & Florack
(86) International application number: PCT/CN2025/074066
(87) International publication number: WO 2026/118171

(57) **Abstract**

The present application provides a method for preparing a make-up powder block having a spot-dyeing effect and a make-up powder block. The method comprises the following steps: preparing a powder base material; preparing a spot-dyeing base material, and mixing the spot-dyeing base material with a water-insoluble coloring material to obtain a spot-dyeing material; applying the at least one spot-dyeing material in dots onto at least one preset position on the bottom of the cavity; filling the mold after dot application with a predetermined amount of the powder base material; freezing and demolding the mixed material within the mold; freeze-drying the demolded powder masterbatch to obtain a make-up powder block having a spot-dyeing effect; wherein the powder base material and the spot-dyeing material exhibit fluidity at normal temperature and pressure. The make-up powder block prepared by the method according to the present application can achieve the spot-dyeing effect of traditional Chinese GongBi painting, with sharp edges in the spot dyed areas. During preparation, the powder block does not develop fine cracks, and exhibits good drop resistance.

## Description

### CROSS REFERENCE TO RELATED APPLICATION

The present application claims priority to Chinese Patent Application No. 202411763231.8, filed on December 2, 2024, entitled "Method for Preparing Make-up powder block Having Spot-dyeing effect and Make-up powder block", the entire contents of which are incorporated herein by reference.

### TECHNICAL FIELD

The present application belongs to the technical field of daily cosmetics, and specifically relates to a method for preparing a make-up powder block having a spot-dyeing effect and a make-up powder block.

### BACKGROUND

Powder cosmetics can modify facial features and enhance the glossiness of makeup. For example, highlighter powder can locally brighten areas, improve facial contours, impart a more radiant finish to the base makeup, eliminate dullness, and create visual contrast through light and shadow, thereby enhancing the three-dimensionality of facial features.

On one hand, from the perspective of functional requirements for make-up powder blocks, there is a need to provide multi-color, color-mixed powder blocks. On the other hand, as the cosmetic industry has developed, product developers have increasingly focused on the emotional value that cosmetic products provide to users. The emotional value of cosmetics is primarily reflected in stimulating positive emotions through aspects such as visual appearance, tactile sensation, and fragrance, thereby influencing the user's psychological state and the effects of skincare and makeup. Consequently, the appearance and color-mixing effect of multi-color mixed make-up powder blocks have also gained importance. However, conventional make-up powder block products currently available on the market are relatively monotonous in appearance, and the patterns and designs of the powder blocks require further development.

### Summary of the present application

In view of this, the present application provides a method for preparing a make-up powder block having a spot-dyeing effect and a make-up powder block, enabling the prepared make-up powder block not only to possess a spot-dyeing effect of traditional Chinese GongBi painting but also to exhibit good drop resistance.

In a first aspect, an embodiment of the present application provides a method for preparing a make-up powder block having a spot-dyeing effect, comprising the following steps: preparing a powder base material: mixing raw materials for preparing the make-up powder block to obtain a powder base material; preparing a spot-dyeing material: preparing at least one spot-dyeing base material, and mixing the spot-dyeing base material with at least one water-insoluble coloring material to obtain at least one spot-dyeing material; wherein the water-insoluble coloring material is selected from water-insoluble pigment powders or lakes; dot application: providing a mold, the mold having at least one cavity with an upward opening, and applying the at least one spot-dyeing material in dots onto at least one preset position on the bottom of the cavity; filling: filling the mold after dot application with a predetermined amount of the powder base material to obtain a mixed material; freeze demolding: freezing the mixed material within the mold to obtain a powder masterbatch, and then separating the powder masterbatch from the mold; freeze-drying: freeze-drying the demolded powder masterbatch to obtain a make-up powder block having a spot-dyeing effect; wherein the powder base material and the spot-dyeing material exhibit fluidity at normal temperature and pressure.

According to an embodiment of the first aspect of the present application, the powder base material and the spot-dyeing material have a viscosity of less than 6000 cP at 25°C; preferably, the powder base material and the spot-dyeing material have a viscosity of 2500 cP to 5000 cP at 25°C.

According to an embodiment of the first aspect of the present application, the powder base material and the spot-dyeing material are composed of the same or different components; preferably, the powder base material and the spot-dyeing material are composed of the same components.

According to an embodiment of the first aspect of the present application, the step of filling the mold after dot application with a predetermined amount of the powder base material comprises: at normal temperature, filling the mold after dot application with the predetermined amount of the powder base material using a pneumatic feeding device.

According to an embodiment of the first aspect of the present application, the pressure in the pneumatic feeding device is from 0.01 to 0.06 MPa, preferably from 0.02 to 0.04 MPa.

According to an embodiment of the first aspect of the present application, the step of freezing the mixed material within the mold is carried out in a liquid nitrogen quick-freezing device, wherein the temperature inside the liquid nitrogen quick-freezing device is from -120°C to -70°C, preferably from -95°C to -80°C.

According to an embodiment of the first aspect of the present application, the freeze-drying comprises pre-freezing, sublimation drying, and desorption drying.

According to an embodiment of the first aspect of the present application, the mold is made of TPR, TPE, silicone, silicone rubber, rubber, or metal. The bottom of the cavity of the mold may have or may not have a three-dimensional pattern design.

According to an embodiment of the first aspect of the present application, the raw materials for preparing the make-up powder block comprise a powder phase component, an oil phase component, and an aqueous phase component, and the raw materials form an oil-in-water emulsion system.

In a second aspect, an embodiment of the present application provides a make-up powder block having a spot-dyeing effect, prepared by any one of the preceding methods. Optionally, the make-up powder block is selected from a pressed powder, a blusher, an eye shadow, or a highlighter.

Compared with the prior art, the present application has at least the following beneficial effects:
in the method provided by the present application, the water-insoluble coloring material is ingeniously utilized in combination with the freeze-drying process to achieve a spot-dyeing effect of traditional Chinese GongBi painting on the surface of the make-up powder block. Specifically, the spot-dyeing color is provided by a water-insoluble pigment powder or lake, which is mixed with the spot-dyeing base material and applied in dots onto the mold, followed by filling with the powder base material. Due to the good fluidity of the powder base material and the spot-dyeing material at normal temperature and pressure, favorable intermixing is achieved at the interface of the materials. Meanwhile, the water-insoluble coloring material is not prone to diffusion within the mixed material, thereby enabling the formation of a localized, irregular spot-dyeing effect on the surface of the make-up powder block that contacts the bottom of the mold cavity and also within its interior. Subsequently, the steps of freeze demolding and freeze-drying are performed to further reduce the cracking rate of the powder block and improve the drop resistance, ultimately obtaining the make-up powder block having a spot-dyeing effect according to the present application.

### BRIEF DESCRIPTION OF THE DRAWINGS

Other features, objectives, and advantages of the present application will become more apparent upon reading the detailed description of non-limiting embodiments with reference to the accompanying drawings, in which the same or similar reference numerals represent the same or similar features.

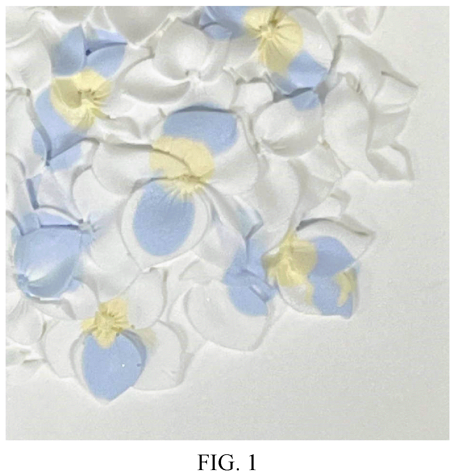

FIG. 1 is a schematic view of the product of Example 1 provided in the present application.

### DETAILED DESCRIPTION

To make the application purpose, technical solutions, and beneficial technical effects of the present application clearer, the present application is further described in detail below with reference to the embodiments. It should be understood that the embodiments described in this specification are only for explaining the present application and are not intended to limit the present application.

For the sake of brevity, only certain ranges are explicitly disclosed herein. However, it should be understood that any lower limit may be combined with any upper limit to recite a range not explicitly recited, as well as, any lower limit may be combined with any other lower limit to recite a range not explicitly recited, in the same way, any upper limit may be combined with any other upper limit to recite a range not explicitly recited. Additionally, although not explicitly recited, every point or individual value within a range is expressly included in the range. Thus, every point or individual value may serve as its own lower or upper limit and be combined with any other point or individual value or any other lower or upper limit, to form a range not explicitly recited.

In the description of the present application, it should be noted that, unless stated otherwise, "above", "below" include the base number, and "more" in "one or more" means two or more.

The above summary of the present application is not intended to describe every disclosed embodiment or every implementation of the present application. The description below more particularly exemplifies exemplary embodiments. Throughout the application, guidance is provided through a series of examples, which may be used in various combinations. In various instances, the listings are merely representative groups and should not be construed as exhaustive.

Spot-dyeing is one of the primary expressive techniques for free-hand flower painting of traditional Chinese GongBi painting, referring to a painting method in which ink or color is directly applied with a writing brush to form spot-dyeing effects. The main characteristics of this method are conception before brushwork, formation upon application, and completion in one continuous stroke, forming irregular fixed-point coloring effects in local parts of the painting.

The inventors of the present application intended to realize the spot-dyeing effect of GongBi painting on the surface of make-up powder. However, surface decoration performed on prepared make-up powder blocks or substantially formed powder masterbatch by means such as inkjet printing or dropwise addition and impregnation of colorants has achieved unsatisfactory effects. For example, inkjet printing can only form a very thin layer of surface decoration on the surface of powder block, which disappears after slight wiping or use. In the method of dropwise addition and impregnation of colorants, colorants do not easily penetrate the surface of powder block and also face the problem that surface decoration disappears after slight wiping or use. If the amount of colorants is increased, uncontrollable peripheral diffusion of colorants occurs, resulting in unclear edges of colored areas, as well as uneven powder block surface, cracking or partial peeling of colored areas.

In view of the above problems, the present application provides a method for preparing a make-up powder block with a spot-dyeing effect, which can prepare a make-up powder block with a spot-dyeing effect, wherein the spot dyed areas have clear edges, the powder block is not prone to cracking, and has good drop resistance.

### Method for Preparing Make-up Powder Block with Spot-dyeing effect

In a first aspect, an embodiment of the present application provides a method for preparing make-up powder block with spot-dyeing effect, comprising the following steps: preparing a powder base material: mixing raw materials for preparing the make-up powder block to obtain a powder base material; preparing a spot-dyeing material: preparing at least one spot-dyeing base material, and mixing the spot-dyeing base material with at least one water-insoluble coloring material to obtain at least one spot-dyeing material; wherein the water-insoluble coloring material is selected from water-insoluble pigment powders or lakes; dot application: providing a mold, the mold having at least one cavity with an upward opening, and applying the at least one spot-dyeing material in dots onto at least one preset position on the bottom of the cavity; filling: filling the mold after dot application with a predetermined amount of the powder base material to obtain a mixed material; freeze demolding: freezing the mixed material within the mold to obtain a powder masterbatch, and then separating the powder masterbatch from the mold; freeze-drying: freeze-drying the demolded powder masterbatch to obtain a make-up powder block having a spot-dyeing effect; wherein the powder base material and the spot-dyeing material exhibit fluidity at normal temperature and pressure.

In the embodiments of the present application, during the preparation of the spot-dyeing material, water-insoluble coloring materials are used, so that pigment migration and diffusion are less likely to occur in the spot-dyeing material, thereby forming local, irregular fixed-point coloring effects on the surface and inside of the make-up powder block in contact with the bottom of the mold cavity. The surface of the make-up powder block in contact with the bottom of the mold cavity is the use surface facing the user.

The embodiments of the present application do not limit the specific types of water-insoluble pigment powders, which can be selected as needed. In some embodiments, the water-insoluble pigment powders are selected from one or more inorganic pigment powders with the following CI numbers: CI 77492, CI 77491, CI 77499, CI 77891, CI 77742, CI 77007, CI 77288, CI 77289, and CI 77510; or the water-insoluble pigment powders are selected from one or more organic pigment powders with the following CI numbers: CI 77266 and CI 15850.

The embodiments of the present application do not limit the specific types of pigment lakes, which can be selected as needed. In some embodiments, the pigment lakes are selected from one or more lakes with the following CI numbers: CI 15850, CI 45380, CI 45410, CI 73360, CI 17200, CI 16035, CI 42090, CI 19140, CI 15985, and CI 47005. Pigment lakes not only maintain particulate and insoluble properties in most solvents to ensure no significant pigment migration and diffusion of colorants in the material, but also have bright hues, high coloring strength and good light fastness, making the formed spot-dyeing colors vivid, flexible, stable and durable.

In the present application, the powder base material and the spot-dyeing material shall have fluidity at normal temperature and pressure. Normal temperature refers to a temperature environment of 20-30°C, and normal pressure refers to the pressure experienced in daily life or workplaces, usually 1013.25 mbar or 760 mmHg. In the practice of the present application, interfacial compatibility between the spot-dyeing material and the powder base material is an important factor affecting the quality of powder block. If the powder base material or the spot-dyeing material tend to solidify before or during filling of the powder base material into the dot-applied mold, poor compatible interfaces are likely to exist between the two materials after filling, increasing the possibility of powder block's cracking. In the present application, by controlling the powder base material and spot-dyeing material to have fluidity at normal temperature and pressure, good intermixing at the contact interface between the powder base material and spot-dyeing material during filling is ensured, interfacial compatibility is improved, and cracking of powder blocks is reduced.

In addition, during drying and water removal of the powder block, if conventional methods such as heating are used for drying, different shrinkage rates may occur during thermal drying due to different compositions of the spot-dyeing material and the powder base material, which also leads to easy cracking between spot dyed areas and the powder base material and poor drop resistance. Meanwhile, evaporation of water during drying is inevitably accompanied by local pigment migration, resulting in unclear edges of spot dyed areas. Therefore, the present application adopts freeze drying for drying powder. Freeze drying is a drying method in which a water-containing material is frozen below its freezing point to convert water into ice, and then the ice is converted into vapor under high vacuum to be removed. Compared with drying methods such as baking, freeze drying can maintain stable material structure and reduce material shrinkage, thereby improving the problems of easy cracking between spot dyed areas and the powder base material and poor drop resistance. In addition, freeze drying can retain the color of raw materials to the greatest extent; the color of spot dyed areas is less prone to migration and loss during drying, and the boundaries of spot dyed areas are relatively clear.

Specifically, in the mixing step, raw materials for preparing the make-up powder block with spot-dyeing effect can be commercially available or self-made. It can be understood that raw materials for preparing the make-up powder block with spot-dyeing effect generally include a powder-phase component, an oil-phase component and an aqueous-phase component. The raw materials can be mixed by various conventional methods in the prior art, such as mechanical stirring, with stepwise addition and uniform mixing. Homogenization, defoaming and other operations can be added during mixing, and different mixing methods and steps can be adopted according to different raw materials, which is not limited in the present application.

When preparing water-insoluble coloring materials, the embodiments of the present application may use the same water-insoluble coloring material, or two or more water-insoluble coloring materials.

The powder base material in the embodiments of the present application may contain other colorants different from the color of coloring materials in spot dyed areas, or may contain no colorants, depending on the desired color of the powder base material outside the spot dyed areas.

The embodiments of the present application do not limit the specific sites of preset positions, which can be selected according to design. Exemplarily, the preset positions are positions with three-dimensional pattern shapes at the bottom of the cavity.

In the filling step of the embodiments of the present application, the predetermined amount can be set according to the volume of the powder cosmetic, mold capacity or filling requirements, and the predetermined amount for each filling can be the same or different.

The preparation method provided in the embodiments of the present application can realize mass production and high production efficiency.

In summary, the method provided in the present application ingeniously utilizes water-insoluble coloring materials combined with a freeze drying process to realize the spot-dyeing effect of traditional Chinese GongBi painting on the surface of make-up powder blocks. Specifically, spot-dyeing colors are provided by water-insoluble pigment powders or lakes, mixed with a spot-dyeing base material and dot-applied onto a mold, followed by filling of the powder base material. Since the powder base material and spot-dyeing material have good fluidity at normal temperature and pressure, good intermixing can be achieved at material's contact surfaces. Meanwhile, water-insoluble coloring materials are less prone to diffusion in the mixed material, so that local, irregular fixed-point coloring effects can be formed on the surface and inside of the make-up powder block in contact with the bottom of the mold cavity. Freezing and demolding as well as freeze drying steps are then performed to further reduce the cracking rate of the powder block and improve the drop resistance of the powder block, finally obtaining the make-up powder block with spot-dyeing effect of the present application.

In some embodiments, the viscosity of the powder base material and the spot-dyeing material at 25°C is less than 6000 cP. In other embodiments, the viscosity of the powder base material and the spot-dyeing material at 25°C is from 2500 cP to 5000 cP, so as to provide good interfacial compatibility between the spot-dyeing material and the powder base material at normal temperature and pressure, ensure good fluidity of both during dot application and filling, enable good intermixing of materials at the two interfaces during filling, and reduce the cracking of powder blocks.

Exemplarily, the viscosity of the powder base material at 25°C can be 6000 cP, 5000 cP, 4500 cP, 4000 cP, 3500 cP, 3000 cP, 2500 cP, 2000 cP, 1500 cP, 1000 cP, or in a range composed of any two of the foregoing values.

Exemplarily, the viscosity of the spot-dyeing material at 25°C can be 6000 cP, 5000 cP, 4500 cP, 4000 cP, 3500 cP, 3000 cP, 2500 cP, 2000 cP, 1500 cP, 1000 cP, or in a range composed of any two of the foregoing values.

In some embodiments, the powder base material and the spot-dyeing material may be composed of the same components or may be composed of different components. When the powder base material and the spot-dyeing material have the same components, the interfacial compatibility between the powder base material and the spot-dyeing material is better, which is beneficial for reducing the cracking of powder blocks.

In some embodiments, the step of filling the mold after dot application with a predetermined amount of the powder base material comprises: at normal temperature, filling the mold after dot application with the predetermined amount of the powder base material using a pneumatic feeding device.

Conventional material filling is performed under heating conditions, for example, maintaining the material's temperature between 40°C and 80°C, so that the material has good fluidity under heating. For the present application, however, a high-temperature environment would increase the local transfer of pigment in the spot dyed areas, affecting the clarity of the edges of the spot dyed areas. Therefore, the present application employs filling under normal temperature conditions, which refers to 20°C to 30°C. Exemplarily, filling is performed at a temperature of 20°C, 22°C, 24°C, 25°C, 26°C, 28°C, or 30°C.

In the embodiments of the present application, a pneumatic feeding device is used during filling. The powder base material is quantitatively filled into the mold via the pneumatic feeding device. The pneumatic feeding assists in filling the mold with the powder base material, avoiding the formation of small bubbles at the corners of fine carved matte patterns on the make-up powder block, and improving the drop resistance of the make-up powder block.

A pneumatic feeding device refers to a device that fills material into a mold by means of pneumatic conveying. Exemplarily, the pneumatic feeding device may include an air pump and a pipeline connected to the air pump, where the air pump provides the required air pressure for conveying, and the pipeline is used for filling the material into the mold. The present application is not limited thereto.

Specifically, the powder base material may be pressed into the mold by the action of air pressure. For example, the pneumatic feeding device includes a feeding pipe, an air pump, and a discharge pipe. The air pump provides the conveying pressure to draw the powder base material from the feeding pipe into the pneumatic feeding device and then press it into the mold through the discharge pipe. Alternatively, the powder base material may be drawn into the mold by the action of air pressure. For example, the pneumatic feeding device is connected to the mold and provides the conveying pressure to draw the powder base material into the mold.

In some embodiments, the pressure in the pneumatic feeding device is from 0.01 to 0.06 MPa, and may be from 0.02 to 0.04 MPa. Exemplarily, the pressure in the pneumatic feeding device may be 0.01 MPa, 0.02 MPa, 0.03 MPa, 0.04 MPa, 0.065 MPa, 0.06 MPa, or in a range composed of any two of the above values.

In some embodiments, the step of solidifying the powder masterbatch at low temperature is carried out in a liquid nitrogen quick-freezing device. Exemplarily, the liquid nitrogen quick-freezing device may be a liquid nitrogen tunnel, a liquid nitrogen freezer, an ultra-low temperature freezer, etc. These devices are commercially available, and the present application is not limited thereto.

After the completion of the filling step in the present application, a powder masterbatch pre-solidified and formed in the mold is obtained. Subsequently, the present application subjects the powder masterbatch to extremely low-temperature solidification using a liquid nitrogen quick-freezing device. The raw material components in the powder masterbatch undergo molecular quick-freezing under extremely low-temperature conditions in the liquid nitrogen quick-freezing device. Cosmetic materials containing components such as oils and emulsions experience a maximum ice crystal formation zone during their freezing and setting process, approximately in the temperature range of -10 to 0°C, where about 80% of the liquid (water, emulsion, etc.) turns into ice. Unlike the ordinary freezing performed before demolding in the prior art, during the molecular quick-freezing process completed within the liquid nitrogen tunnel in the present application, the powder masterbatch rapidly passes through the maximum ice crystal formation zone. The ice crystals are distributed more finely and uniformly within the powder masterbatch. In the subsequent freeze-drying step, these fine and uniform ice crystals are directly converted into vapor and removed, further enhancing the drop resistance of the freeze-dried powder blocks.

In some embodiments, the liquid nitrogen quick-freezing device is a liquid nitrogen tunnel, and the temperature inside the liquid nitrogen tunnel is from -120°C to -70°C, preferably from -95°C to -80°C. The liquid nitrogen tunnel, also referred to as a liquid nitrogen tunnel freezer, is a device that uses liquid nitrogen as a cold source and employs a continuous production method, wherein materials are fed into the freezing tunnel via a conveyor belt to achieve continuous material input/output and rapid freezing. Exemplarily, the powder masterbatch passes through the liquid nitrogen tunnel on a conveyor belt within the liquid nitrogen tunnel at a speed of 0.4 to 1.2 m/min, and the time for the powder masterbatch passing through the liquid nitrogen tunnel is from 6 minutes to 10 minutes.

In some embodiments, the freeze-drying is carried out in a freeze-drying apparatus.

In some embodiments, the freeze-drying comprises pre-freezing, sublimation drying, and desorption drying. Exemplarily, the freeze-drying may employ the following steps: pre-freezing is carried out at a temperature of -50°C to -40°C for a pre-freezing time of 0.3 to 0.5 hours; sublimation drying comprises: subjecting the powder masterbatch to a temperature increase treatment to a temperature ranging from -3°C to 3°C, the temperature increase treatment comprising a plurality of first temperature increase stages and a plurality of first holding stages, wherein the first temperature increase stages and the first holding stages are carried out alternately, each first temperature increase stage involves a temperature increase of 0.5 to 1°C/min for 10 to 20 min, and the time for the first holding stage is 1.5 to 2 hours; desorption drying comprises: subjecting the solidified powder masterbatch to a drying treatment at a temperature of 20°C to 60°C, the drying treatment comprising a plurality of second temperature increase stages and a plurality of second holding stages, wherein the second temperature increase stages and the second holding stages are carried out alternately, each second temperature increase stage involves a temperature increase of 0.5 to 1°C/min for 10 to 20 min, and the time for the second holding stage is 1.5 to 2 hours. The process parameters for freeze-drying can be adjusted during actual production, and the present application is not limited thereto.

Sublimation drying in the present application can be understood as: a drying method in which the material to be dried is rapidly frozen, and then under high vacuum conditions, the ice therein is sublimated into water vapor and removed. Desorption drying in the present application can be understood as: a drying method in which the material to be dried is placed under negative pressure or vacuum conditions, and is appropriately heated to reach the boiling point under negative pressure, or is cooled to solidify the material, after which the material is dried by controlling the melting point. In some embodiments of the present application, pre-freezing may be omitted, and sublimation drying and desorption drying may be performed directly, or only sublimation drying may be performed.

In some embodiments, the solvent content in the prepared make-up powder block is less than 5%, preferably within 1%.

In some embodiments, the mold is made of TPR, TPE, silicone, silicone rubber, rubber, or metal. The bottom of the cavity of the mold may have or may not have a three-dimensional pattern design.

The preparation method in the embodiments of the present application can employ molds made of the aforementioned TPR, TPE, silicone, silicone rubber, rubber, or metal, all of which can produce powder cosmetics having a spot-dyeing effect.

The bottom of the cavity of the mold used may be flat, without a three-dimensional pattern design; or the bottom of the cavity of the mold may have a three-dimensional pattern design, which can be diverse. Exemplarily, the three-dimensional pattern design may be a floral pattern, but the embodiments of the present application are not limited thereto.

In some embodiments, the spot-dyeing effect is formed on the contact surface between the make-up powder block and the bottom of the cavity, and extends towards the interior of the make-up powder block. The extension distance can be adjusted by controlling the amount of spot-dyeing material used.

In some embodiments, the raw materials for preparing the make-up powder block include a powder phase component, an oil phase component, and an aqueous phase component, the raw materials forming an oil-in-water emulsion system.

In some embodiments, the powder phase component comprises one or more of a filler and a colorant.

The oil phase component comprises one or more of a moisturizing and emollient agent, an antioxidant, and a sunscreen agent.

The aqueous phase component comprises one or more of a solvent, a thickener, a film-forming agent, a moisturizing and emollient agent, an emulsifier, a preservative, and an active substance.

In the embodiments of the present application, the raw materials form an oil-in-water emulsion system. The oil-in-water emulsion system has water as the continuous phase, which allows the water-soluble pigment to flow better, resulting in a more natural gradient effect.

In some embodiments, the thickener and the film-forming agent are selected from natural water-soluble polymers or derivatives thereof, synthetic water-soluble polymers, or water-based inorganic thickeners.

Natural water-soluble polymers or derivatives thereof are selected from at least one of alginic acid, agar, carrageenan, Xanthan, gellan gum, chondrus crispus, xanthan gum, guar gum, tamarind gum, tara gum, gum arabic, tragacanth gum, Peruvian carob, pectin, arabinogalactan, wheat protein, soy protein, gelatin, casein, chitosan, curdlan polysaccharide, cyclodextrin, hyaluronic acid, konjac glucomannan, tremella polysaccharide, curdlan, microcrystalline cellulose, methyl cellulose, hydroxyethyl cellulose, hydroxypropyl cellulose, starch phosphate, starch, sodium starch hydroxypropylate, sodium polyacrylate grafted starch, and hydroxypropyl guar gum.

Synthetic water-soluble polymers are selected from at least one of acrylates/C10-30 alkyl acrylate crosspolymer, carbomer, acrylates/octylacrylamide copolymer, acrylates/ethylhexyl acrylate copolymer, sodium acrylate/sodium acryloyldimethyl taurate copolymer, acrylates/octylacrylamide copolymer, styrene/acrylates copolymer, acrylates copolymer, hydroxyethyl acrylate/sodium acryloyldimethyl taurate copolymer, polyacrylate crosspolymer-6, acrylates/steareth-20 methacrylate copolymer, ammonium acryloyldimethyltaurate/beheneth-25 methacrylate crosspolymer, ammonium acrylates copolymer, sodium polyacrylate, sodium polyacryloyldimethyl taurate, polyacrylamide, polyvinylpyrrolidone, polyvinyl alcohol, VP/VA copolymer, polyurethane-1, and polyurethane-35.

Water-based inorganic thickeners are selected from at least one of magnesium aluminum silicate, lithium magnesium sodium silicate, magnesium lithium silicate, sodium magnesium silicate, montmorillonite or derivatives thereof.

In some embodiments, the moisturizing and emollient agent is selected from at least one of alcohols, silicone oils, mineral oils, synthetic oils, animal and vegetable oils or derivatives thereof.

Animal and vegetable oils or derivatives thereof are selected from at least one of glycerin, propylene glycol, dipropylene glycol, pentylene glycol, butylene glycol, ethylene glycol, hexylene glycol, sorbitol, xylitol, polyethylene glycol, dimethicone, caprylyl methicone, phenyl trimethicone, cetyl dimethicone, C30-45 alkyl dimethicone/polypropylsilsesquioxane crosspolymer, bis-PEG-18 methyl ether dimethyl silane, white mineral oil, petrolatum, hydrogenated polyisobutene, lanolin and derivatives thereof, octyldodecanol, caprylic/capric triglyceride, ethylhexyl palmitate, bis-diglyceryl polyacyladipate-2, isononyl isononanoate, jojoba seed oil, coconut oil, hydrogenated coconut glycerides, meadowfoam seed oil, olive oil, squalane, C9-12 alkane, coco-caprylate/caprate, tocopheryl acetate, and methyl gluceth-10.

In some embodiments, the filler is selected from at least one of mica, talc, synthetic fluorphlogopite, kaolin, bentonite, boron nitride, bismuth oxychloride, silica, potassium sodium aluminum silicate, lauroyl lysine, magnesium stearate, magnesium myristate, calcium aluminum borosilicate, tin oxide, sodium calcium borosilicate, aluminum hydroxide, zinc stearate, starch and derivatives thereof, or plastic microspheres.

In some embodiments, the colorant is selected from at least one of iron oxides (CI 77499, CI 77491, CI 77492), titanium dioxide (CI 77891), iron blue (CI 77510), ultramarine (CI 77007), manganese violet (CI 77742), chromium oxide green (CI 77288), chromium hydroxide green, carmine (CI 75470), carbon black (CI 77266), CI 15850, CI 19140, CI 45410, CI 16035, CI 45380, CI 17200, CI 73360, CI 42090, and CI 47005.

In some embodiments, the preservative is selected from at least one of phenoxyethanol, parabens, chlorphenesin, potassium sorbate, sodium benzoate, or preservative enhancers

Preservative enhancers are selected from at least one of caprylyl glycol, hydroxyacetophenone, ethylhexylglycerin, 1,2-hexanediol, caprylhydroxamic acid, 1,2-pentanediol, glyceryl caprylate, or p-cymene.

### Make-up powder block Having Spot-dyeing effect

In a second aspect, an embodiment of the present application provides a make-up powder block having a spot-dyeing effect, prepared by any one of the preceding methods. Optionally, the powder cosmetic is selected from a pressed powder, a blusher, an eye shadow, or a highlighter powder.

The make-up powder block prepared by the embodiments of the present application has a favorable spot-dyeing effect, with clear edges of the spot dyed areas. The powder block is not prone to cracking and has good drop resistance.

In some embodiments, the make-up powder block having a spot-dyeing effect is selected from a pressed powder, a blusher, an eye shadow, or a highlighter powder. The powder cosmetic having a spot-dyeing effect may also include other functional products.

### Examples

The following examples describe the disclosure of the present application in more detail. These examples are provided for illustrative purposes only, as various modifications and variations within the scope of the disclosure of the present application will be apparent to those skilled in the art. Unless otherwise stated, all parts, percentages, and ratios reported in the following examples are based on mass, and all reagents used in the examples are commercially available or synthesized according to conventional methods, and can be used directly without further processing. Furthermore, the instruments used in the examples are commercially available.

### Example 1

(1) Preparation of powder base material: the following raw materials were used: Phase A: water; Phase B: glycerin, Chondrus crispus; Phase C: potassium chloride; Phase D: polysorbate-60, cetearyl ethylhexanoate, squalane, octyldodecyl stearoyloxystearate, tocopheryl acetate; Phase E: silica, synthetic fluorphlogopite; Phase F: pigment powder; Phase G: chlorphenesin, caprylyl glycol. Phase B was pre-mixed first. After heating Phase A, Phase B, Phase C, and Phase D were added to Phase A, followed by emulsification and homogenization. Phase E and Phase F were mixed with stirring. Finally, Phase G was added, and the mixture was emulsified and homogenized. The emulsified and homogenized material was subjected to vacuum defoaming to obtain a powder base material, which exhibited good fluidity at 25°C and normal pressure.
(2) Preparation of spot-dyeing material: A spot-dyeing base material was prepared. The composition and preparation steps of the spot-dyeing base material in this example were the same as those of the powder base material. Therefore, it could be prepared separately, or a portion could be taken from the powder base material prepared in step (1). Two water-insoluble coloring materials used in this example were as follows: Pigment 1 - trade name UNIPURE BLUE LC680 (INCI: CI77007) and Pigment 2 - trade name PRIMROSE-YELLOW (INCI: CI77492). The spot-dyeing base material was mixed with the above two water-insoluble pigment powders, respectively, to obtain the spot-dyeing materials.
(3) Dot application: A silicone mold was provided, which had a cavity with an upward opening. The bottom of the cavity had a three-dimensional floral carved matte pattern. According to the predetermined spot-dyeing design, the two spot-dyeing materials were applied in dots onto several preset positions on the bottom of the cavity of the silicone mold.
(4) Filling: After dot application, the powder base material was heated to 60°C and filled into the mold with a predetermined amount by gravity filling via a filling machine.
(5) Freeze demolding: The mold filled with the mixed material was placed in a freezer at -15°C for freezing to obtain a powder masterbatch. Then, the powder masterbatch was separated from the mold.
(6) Freeze-drying: The demolded powder masterbatch was freeze-dried to obtain a make-up powder block having a spot-dyeing effect. The freeze-drying comprised pre-freezing, sublimation drying, and desorption drying. Pre-freezing was carried out at -50°C to -40°C for a pre-freezing time of 0.3 to 0.5 hours. Sublimation drying comprised: subjecting the powder masterbatch to a temperature increase treatment to a temperature ranging from -3°C to 3°C, the temperature increase treatment comprising a plurality of first temperature increase stages and a plurality of first holding stages, wherein the first temperature increase stages and the first holding stages were carried out alternately. Desorption drying comprised: subjecting the solidified powder masterbatch to a drying treatment at 20°C to 60°C, the drying treatment comprising a plurality of second temperature increase stages and a plurality of second holding stages, wherein the second temperature increase stages and the second holding stages were carried out alternately.

### Example 2

Example 2 differed from Example 1 in that the water-insoluble coloring materials used were different.

### Examples 3-5

Examples 3-5 differed from Example 1 in that the viscosities of the powder base material and the spot-dyeing material were different.

### Examples 6-7

Examples 6-7 differed from Example 1 in that instead of heating the material before filling, the material was filled into the mold with a predetermined amount using a pneumatic feeding device at normal temperature after dot application of the water-insoluble coloring material. Specifically, the material was pressed into the mold by the action of air pressure. In Examples 6 and 7, the pressures in the pneumatic feeding device were 0.02 MPa and 0.06 MPa, respectively.

### Examples 8-9

Examples 8-9 differed from Example 1 in that the step of freezing the mixed material within the mold was carried out in a liquid nitrogen tunnel. The powder masterbatch passed through the liquid nitrogen tunnel on a conveyor belt at a speed of 0.6 m/min, and the residence time of the powder masterbatch within the liquid nitrogen tunnel was 10 minutes. In Examples 8 and 9, the temperatures inside the liquid nitrogen tunnel were from -80°C and -95°C, respectively.

### Example 10

Example 10 differed from Example 1 in that the material was pressed into the mold by the action of air pressure, with the pressure in the pneumatic feeding device being 0.04 MPa; and the step of freezing the mixed material within the mold was carried out in a liquid nitrogen tunnel, with the temperature inside the liquid nitrogen tunnel being -85°C.

### Comparative Examples 1-4

Comparative Example 1 differed from Example 1 in that the drying step (6) did not employ freeze-drying but instead employed drying in an oven at 55°C.

Comparative Example 2 differed from Example 1 in that the drying step (6) did not employ freeze-drying but instead employed drying in an oven at 40°C.

Comparative Example 3 differed from Example 1 in that the powder base material and the spot-dyeing material had a viscosity of about 8000 cP at normal temperature and pressure, exhibiting poor fluidity.

Comparative Example 4 differed from Example 1 in that the compact base and the spot-dyeing material had a viscosity of about 8000 cP at normal temperature and pressure, exhibiting poor fluidity. During the preparation process, normal-temperature pneumatic filling was employed, and ultra-low temperature freezing in a liquid nitrogen tunnel was performed before demolding.

The raw materials and preparation process parameters for Examples 1-10 and Comparative Examples 1-4 are shown in Table 1.

**Table 1 Process parameters for Examples 1-10 and Comparative Examples 1-4**

| | Powder based material and spot-dyeing material | | Coloring material | Filling | | | Freeze Demolding | | Drying |
|---|---|---|---|---|---|---|---|---|---|
| | Fluidity at normal temperature and pressure | Viscosity at 25°C | Coloring material | Filling method | Filling temperat ure | Filling pressure | Freezing equipment | Freezing temperature | Drying method |
| Example 1 | Having fluidity | 3000cP | Pigment powder I77007 | Heat-assisted filling | 60°C | Normal pressure | Freezer | -15°C | Freeze-drying |
| | | | Pigment powder I77492 | | | | | | |
| Example 2 | Having fluidity | 3000cP | Pigment lake I45410 | Heat-assisted filling | 60°C | Normal pressure | Freezer | -15°C | Freeze-drying |
| | | | Pigment lake I73360 | | | | | | |
| Example 3 | Having fluidity | 2500cP | Pigment powder I77007 | Heat-assisted filling | 60°C | Normal pressure | Freezer | -15°C | Freeze-drying |
| | | | Pigment powder I77492 | | | | | | |
| Example 4 | Having fluidity | 4000cP | Pigment powder I77007 | Heat-assisted filling | 60°C | Normal pressure | Freezer | -15°C | Freeze-drying |
| | | | Pigment powder I77492 | | | | | | |
| Example 5 | Having fluidity | 5000cP | Pigment powder I77007 | Heat-assisted filling | 60°C | Normal pressure | Freezer | -15°C | Freeze-drying |
| | | | Pigment powder I77492 | | | | | | |
| Example 6 | Having fluidity | 3000cP | Pigment powder I77007 | Pneumat ic filling | Normal temperat ure | 0.02MPa | Freezer | -15°C | Freeze-drying |
| | | | Pigment powder I77492 | | | | | | |
| Example 7 | Having fluidity | 3000cP | Pigment powder I77007 | Pneumat ic filling | Normal temperat ure | 0.06MPa | Freezer | -15°C | Freeze-drying |
| | | | Pigment powder I77492 | | | | | | |
| Example 8 | Having fluidity | 3000cP | Pigment powder I77007 | Heat-assisted filling | 60°C | Normal pressure | Liquid nitrogen tunnel | -80°C | Freeze-drying |
| | | | Pigment powder I77492 | | | | | | |
| Example 9 | Having fluidity | 3000cP | Pigment powder I77007 | Heat-assisted filling | 60°C | Normal pressure | Liquid nitrogen tunnel | -95°C | Freeze-drying |
| | | | Pigment powder I77492 | | | | | | |
| Example 10 | Having fluidity | 3000cP | Pigment powder I77007 | Pneumat ic filling | Normal temperat ure | 0.04MPa | Liquid nitrogen tunnel | -85°C | Freeze-drying |
| | | | Pigment powder I77492 | | | | | | |
| Comparative Example 1 | Having fluidity | 3000cP | Pigment powder I77007 | Heat-assisted filling | 60°C | Normal pressure | Freezer | -15°C | 55°C 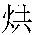 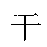 |
| | | | Pigment powder I77492 | | | | | | |
| Comparative Example 2 | Having fluidity | 3000cP | Pigment powder I77007 | Heat-assisted filling | 60°C | Normal pressure | Freezer | -15°C | 40°C 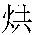 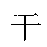 |
| | | | Pigment powder I77492 | | | | | | |
| Comparative Example 3 | Poor fluidity | 8000cP | Pigment powder I77007 | Heat-assisted filling | 60°C | Normal pressure | Freezer | -15°C | Freeze-drying |
| | | | Pigment powder I77492 | | | | | | |
| Comparative Example 4 | Poor fluidity | 8000cP | Pigment powder I77007 | Pneumat ic filling | Normal temper ature | 0.04M Pa | Liquid nitrogen tunnel | -85°C | Freeze-drying |
| | | | Pigment powder I77492 | | | | | | |

### Test Methods

Powder cakes were prepared according to the methods of Examples 1-10 and Comparative Examples 1-4 with 100 powder cakes prepared for each example or comparative example. Appearance observation and drop tests were conducted.

### 1) Appearance Observation

The appearance of the powder cakes was observed with the naked eye or with the aid of a microscope, including observation of the spot-dyeing effect. The observation focused on the spot-dyeing effect of the powder cake, specifically whether the edges of the dyed areas were sharp, whether color loss occurred, whether there were bubbles at the sharp parts of the fine carved matte surface, and whether the powder cake exhibited cracking.

The recorded appearance was based on the observation results of more than 90% of the 100 powder cakes. The percentage of powder cakes exhibiting micro-cracking was calculated as the proportion of the 100 powder cakes that showed micro-cracking.

### 2) Drop Test

The rotating screws of the drop test stand were adjusted to set the flat plate at a height of 30 cm. The powder block was placed in the center position of the test height, with its front side facing upward.

The first drop test was performed as followed: the button of the test stand was pressed to drop the powder block onto the rigid plate. The powder block that had fallen onto the table surface was picked up and inspected for any cracks or breakage on its surface. If the powder block cracked or broke, it was excluded from further drop testing. If the powder block showed no abnormality, the second drop test was conducted. The operational steps for the second drop test were the same as those for the first drop test. If the powder block cracked or broke, it was excluded from further drop testing. If the powder block showed no abnormality, the third drop test was conducted. The operational steps for the third drop test were the same as those for the first drop test.

The ratio of the number of broken powder block in each drop test to the total number of powder block subjected to that drop test was the breakage rate for that drop test. The first, second, and third breakage rates for Examples 1-10 and Comparative Examples 1-4 were recorded, respectively. The drop resistance score was determined based on the sum of the three breakage rates. The scoring criteria for drop resistance are shown in Table 2.

**Table 2 Scoring Criteria for Drop Resistance**

| Sum of breakage rates for 3 drops | Less than 5% | 5%~10% | 10%~15% | 15%~20% | 20%~25% | 25%~30% |
|---|---|---|---|---|---|---|
| Drop resistance score | 5 points | 4 points | 3 points | 2 points | 1 point | 0 point |

The above test results for Examples 1-10 and Comparative Examples 1-4 were recorded in Table 3.

**Table 3 Test Results for Examples 1-10 and Comparative Examples 1-4**

| | Observation of spot-dyeing effect | | | Drop Resistance Score After Preparation |
|---|---|---|---|---|
| | Whether the edges of the spot dyed area are sharp; whether color loss occurs before and after drying | Whether bubbles are present at sharp details of the finely carved matte surface | Percentage of micro-cracking appearing on the powder block | |
| Example 1 | Edges relatively sharp; no color loss | Slight bubbles at sharp details of the finely carved matte surface, acceptable | About 5% | 3 |
| Example 2 | Edges relatively sharp; no color loss | Slight bubbles at sharp details of the finely carved matte surface, acceptable | About 5% | 3 |
| Example 3 | Edges relatively sharp; no color loss | Slight bubbles at sharp details of the finely carved matte surface, acceptable | About 5% | 3 |
| Example 4 | Edges relatively sharp; no color loss | Slight bubbles at sharp details of the finely carved matte surface, acceptable | About 5% | 3 |
| Example 5 | Edges relatively sharp; no color loss | Slight bubbles at sharp details of the finely carved matte surface, acceptable | About 5% | 3 |
| Example 6 | Edges very sharp; no color loss | No bubbles, filling of finely carved matte surface perfect | About 3% | 4 |
| Example 7 | Edges very sharp; no color loss | No bubbles, filling of finely carved matte surface perfect | About 3% | 4 |
| Example 8 | Edges relatively sharp; no color loss | Slight bubbles at sharp details of the finely carved matte surface, acceptable | Less than 2% | 5 |
| Example 9 | Edges relatively sharp; no color loss | Slight bubbles at sharp details of the finely carved matte surface, acceptable | Less than 2% | 5 |
| Example 10 | Edges very sharp; no color loss | No bubbles, filling of finely carved matte surface perfect | Less than 2% | 5 |
| Comparative Example 1 | Edges not sharp, color loss observed before and after drying | Bubbles visible at sharp parts of the finely carved matte surface | 8%~10% | 1 |
| Comparative Example 2 | Edges not sharp, color loss observed before and after drying | Bubbles visible at sharp parts of the finely carved matte surface | 8%~10% | 1 |
| Comparative Example 3 | Edges sharp, no color loss | Bubbles visible at sharp parts of the finely carved matte surface | More than 20% | 0 |
| Comparative Example 4 | Edges sharp, no color loss | Bubbles visible at sharp parts of the finely carved matte surface | More than 20% | 0 |

As shown in the test results in Table 3, Examples 1 to 10 adopted the method for preparing a make-up powder block with a spot-dyeing effect provided by the present application. After preparing the powder base material and the spot-dyeing material, through dot application, filling, freeze demolding, and freeze-drying, the prepared make-up powder block exhibited better spot-dyeing effects, improved edge definition in the spot dyed areas, better filling of intricately carved surfaces, and reduced occurrence of cracking and breakage upon dropping, compared with the make-up powder block of Comparative Examples 1 to 4, which were not prepared using the method of the present application.

In Examples 1 to 10, Examples 1 to 5 used a spot-dyeing material comprising a water-insoluble coloring material for spot-dyeing. By controlling the powder base material and the spot-dyeing material to have good fluidity at normal temperature and pressure, their fluidity under such conditions was ensured, thereby improving the interfacial compatibility between the spot-dyeing material and the powder base material. Meanwhile, a freeze-drying process was employed to dry the powder block, further reducing potential differences in shrinkage between different materials during drying, resulting in a reduced cracking rate and significantly improved drop resistance of the prepared make-up powder block. Additionally, the freeze-drying process also ensured that the color of the powder block was less prone to migration and loss during drying, making the spot-dyeing color of the prepared make-up powder block easier to control and achieving sharper edges in the dyed areas. For the product effect of Example 1, please refer to FIG. 1.

Examples 6 and 7 further improved the filling method based on Examples 1 to 5, using a pneumatic feeding device for material filling. Compared with the heat-assisted filling used in Examples 1 to 5, the edges of the dyed areas were sharper, the filling of fine carved matte surfaces was more perfect, no bubbles were generated in sharp parts, and due to the auxiliary effect of air pressure on material filling, the rates of micro-cracking and breakage upon dropping during the preparation process of powder blocks were further reduced.

Examples 8 and 9 further improved the freezing method before demolding based on Examples 1 to 5, using a liquid nitrogen tunnel for ultra-low temperature pre-freezing of the mixed material before demolding. Compared with the conventional freezing method used in Examples 1 to 5, the ultra-low temperature freezing in the liquid nitrogen tunnel allowed fine, uniformly distributed ice crystals to form inside the material in a very short time. After these fine, uniformly distributed ice crystals were directly sublimated and removed as water vapor through the freeze-drying step, the drop resistance of the prepared powder block was further enhanced.

Example 10, based on Examples 1 to 5, used a pneumatic feeding device for material filling and employed a liquid nitrogen tunnel for ultra-low temperature pre-freezing of the mixed material before demolding. Consequently, the edges of the dyed areas were very sharp, there was no color loss during drying, the cracking rate of the cosmetic powder cake was reduced to below 2%, and the drop resistance score reached 5 points.

The drying steps in Comparative Examples 1 and 2 did not use freeze-drying; instead, drying was carried out at 55°C and 40°C using oven drying. The edges of the dyed areas were unclear, color loss occurred before and after drying, micro-cracks appeared between the dyed areas and the powder cake matrix during the drying process, and the drop resistance score was low.

In Comparative Examples 3 and 4, the viscosity of the powder base material and the spot-dyeing material reached 8000 cP at normal temperature and pressure, resulting in poor fluidity under such conditions. During filling, both materials tended to be in a solidified state and could not mix well, leading to poor interfacial compatibility. Micro-cracks were prone to appear between the powder base material and the dyed areas during drying, and the drop resistance of the prepared make-up powder block was also poor. Even when pneumatic filling at normal temperature was employed during the preparation process and liquid nitrogen tunnel ultra-low temperature freezing was applied before demolding, the drop resistance of the make-up powder block could not be significantly improved.

Described above are merely specific implementations of the present application, but the protection scope of the present application is not limited thereto. Any skilled person who is familiar with this art would readily conceive of various equivalent modifications or substitutions within the technical scope of the disclosure of the present application, and these modifications or substitutions shall fall within the protection scope of the present application. Therefore, the protection scope of the present application shall be subject to the protection scope of the claims.

## Claims

1. A method for preparing a make-up powder block having a spot-dyeing effect, comprising the following steps:
preparing a powder base material: mixing raw materials for preparing the make-up powder block to obtain a powder base material;
preparing a spot-dyeing material: preparing at least one spot-dyeing base material, and mixing the spot-dyeing base material with at least one water-insoluble coloring material to obtain at least one spot-dyeing material; wherein the water-insoluble coloring material is selected from water-insoluble pigment powders or lakes;
dot application: providing a mold, the mold having at least one cavity with an upward opening, and applying the at least one spot-dyeing material in dots onto at least one preset position on the bottom of the cavity;
filling: filling the mold after dot application with a predetermined amount of the powder base material to obtain a mixed material;
freeze demolding: freezing the mixed material within the mold to obtain a powder masterbatch, and then separating the powder masterbatch from the mold;
freeze-drying: freeze-drying the demolded powder masterbatch to obtain a make-up powder block having a spot-dyeing effect;
wherein the powder base material and the spot-dyeing material exhibit fluidity at normal temperature and pressure.

2. The method for preparing a make-up powder block having a spot-dyeing effect according to claim 1, wherein the powder base material and the spot-dyeing material have a viscosity of less than 6000 cP at 25°C; preferably, the powder base material and the spot-dyeing material have a viscosity of 2500 cP to 5000 cP at 25°C.

3. The method for preparing a make-up powder block having a spot-dyeing effect according to claim 1, wherein the powder base material and the spot-dyeing material are composed of the same or different components; preferably, the powder base material and the spot-dyeing material are composed of the same components.

4. The method for preparing a make-up powder block having a spot-dyeing effect according to claim 1, wherein the step of filling the mold after dot application with a predetermined amount of the powder base material comprises: at normal temperature, filling the mold after dot application with the predetermined amount of the powder base material using a pneumatic feeding device.

5. The method for preparing a make-up powder block having a spot-dyeing effect according to claim 4, wherein a pressure in the pneumatic feeding device is from 0.01 to 0.06 MPa, preferably from 0.02 to 0.04 MPa.

6. The method for preparing a make-up powder block having a spot-dyeing effect according to claim 1,wherein the step of freezing the mixed material within the mold is carried out in a liquid nitrogen quick-freezing device, wherein a temperature inside the liquid nitrogen quick-freezing device is from -120°C to -70°C, preferably from -95°C to -80°C.

7. The method for preparing a make-up powder block having a spot-dyeing effect according to claim 1, wherein the freeze-drying comprises pre-freezing, sublimation drying, and desorption drying.

8. The method for preparing a make-up powder block having a spot-dyeing effect according to claim 1, wherein the mold is made of TPR, TPE, silicone, silicone rubber, rubber, or metal; and a bottom of the cavity of the mold may have or may not have a three-dimensional pattern design.

9. The method for preparing a make-up powder block having a spot-dyeing effect according to claim 1, wherein the raw materials for preparing the make-up powder block comprise a powder phase component, an oil phase component, and an aqueous phase component, and the raw materials form an oil-in-water emulsion system.

10. A make-up powder block having a spot-dyeing effect, prepared by the method according to any one of claims 1 to 9.
